(19) Europäisches Patentamt
European Patent Office
Office européen des brevets

(11) Veröffentlichungsnummer: **0 490 228 A1**

(12) # EUROPÄISCHE PATENTANMELDUNG

(21) Anmeldenummer: **91120734.8**

(51) Int. Cl.5: **C07C 229/16**, C07C 227/26

(22) Anmeldetag: **03.12.91**

(30) Priorität: **14.12.90 DE 4039922**

(43) Veröffentlichungstag der Anmeldung:
**17.06.92 Patentblatt 92/25**

(84) Benannte Vertragsstaaten:
**BE DE ES FR GB IT NL SE**

(71) Anmelder: **BASF Aktiengesellschaft**
**Carl-Bosch-Strasse 38**
**W-6700 Ludwigshafen(DE)**

(72) Erfinder: **Zipplies, Matthias, Dr.**
**Eichstrasse 36**
**W-6730 Neustadt(DE)**
Erfinder: **Braun, Gerold, Dr.**
**Pruemer Strasse**
**W-6700 Ludwigshafen(DE)**
Erfinder: **Houben, Jochen, Dr.**
**Hahnenweide 21**
**W-4154 Toenisvorst 2(DE)**
Erfinder: **Mueller, Josef, Dr.**
**Rieslingstrasse 15**
**W-6711 Grosskarlbach(DE)**
Erfinder: **Schneider, Rolf, Dr.**
**Feldbergstrasse 21**
**W-6800 Mannheim 1(DE)**
Erfinder: **Wild, Jochen, Dr.**
**St.-Martin-Strasse 22**
**W-6701 Ruppertsberg(DE)**
Erfinder: **Widder, Rudi, Dr.**
**In der Taesch 7**
**W-6906 Leimen(DE)**

(54) **Verfahren zur Herstellung von beta-Alanindiessigsäure und ihren Alkalimetallsalzen.**

(57) Herstellung von β-Alanindiessigsäure und ihren Alkalimetallsalzen I

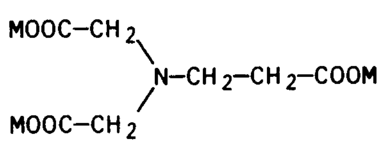

I

in der die Variablen M unabhängig voneinander für Wasserstoff oder Alkalimetall stehen, wobei auch eine Variable M für Ammonium stehen kann, indem man β-Alanin oder ein Alkalimetallsalz oder das Ammoniumsalz hiervon mit Alkalimetallcyanid und Formaldehyd in wäßrig-alkalischem Medium umsetzt und für den Fall M = Wasserstoff die Carboxylgruppen durch Zugabe von Mineralsäure aus ihren Salzen freisetzt.

EP 0 490 228 A1

Die vorliegende Erfindung betrifft ein verbessertes Verfahren zur Herstellung von β-Alanindiessigsäure und ihren Alkalimetallsalzen der Formel I

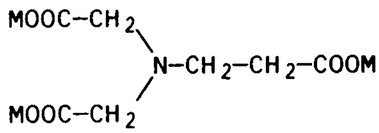

I

in der die Variablen M unabhängig voneinander für Wasserstoff oder Alkalimetall stehen, wobei auch eine Variable M für Ammonium stehen kann.

Weiterhin betrifft die Erfindung die Verwendung der erfindungsgemäß hergestellten Verbindungen I als Komplexbildner.

Aus der DE-A-38 29 859 (1) ist ein Herstellungsverfahren für β-Alanindiessigsäure und deren Alkalimetall- und Ammoniumsalzen bekannt, bei dem man Iminodiessigsäure und Acrylsäure miteinander umsetzt. Die so erhaltenen Produkte haben Reinheiten von 97 bis 99,5 Gew.-%.

Weiterhin ist aus der Literaturstelle V.G. Yashunskii et al., Zh. Vses. Khim. Obschestva im. D.I. Mendeleeva 10 (1965), S. 105 - 106, (2), zitiert in Chem. Abstr. 62 (1965), 16232a, bekannt, daß man β-Alanin zu β-Alanindiacetonitril cyanomethylieren kann.

Da β-Alanindiessigsäure und ihre Salze hauptsächlich als Komplexbildner, beispielsweise im Wasch- und Reinigungsmittelsektor oder in der Photoindustrie, Verwendung finden, sind hohe Reinheiten der Verbindungen I erwünscht. Insbesondere beim Einsatz in der Photoindustrie sind Produkte mit einer Reinheit bis zu 99,5 Gew.-%, wie sie gemäß (1) erhalten werden können, noch nicht ausreichend sauber.

Der vorliegenden Erfindung lag daher die Aufgabe zugrunde, die Verbindungen I durch ein einfaches und wirtschaftliches Verfahren ohne spezielle Reinigungsschritte mit einer Reinheit von mehr als 99,5 Gew.-% herzustellen.

Demgemäß wurde ein Verfahren zur Herstellung von β-Alanindiessigsäure und ihren Alkalimetallsalzen der Formel I

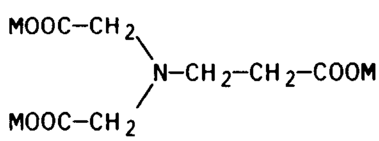

I

in der die Variablen M unabhängig voneinander für Wasserstoff oder Alkalimetall stehen, wobei auch eine Variable M für Ammonium stehen kann, gefunden, welches dadurch gekennzeichnet ist, daß man β-Alanin oder ein Alkalimetallsalz oder das Ammoniumsalz hiervon mit Alkalimetallcyanid und Formaldehyd in wäßrig-alkalischem Medium umsetzt und für den Fall M = Wasserstoff die Carboxylgruppen durch Zugabe von Mineralsäure aus ihren Salzen freisetzt.

Steht M für Alkalimetall, sind hiermit vor allem Natrium und Kalium gemeint.

Das erfindungsgemäße Verfahren betrifft vor allem die Herstellung von Alkalimetallsalzen I, bei denen alle drei Variablen M für das gleiche Alkalimetall stehen, sowie insbesondere die Herstellung der freien β-Alanindiessigsäure, bei der alle drei Variablen M Wasserstoff bedeuten. Daneben können nach dem erfindungsgemäßen Verfahren aber auch gemischte Salze I mit verschiedenen Alkalimetallen und gegebenenfalls Ammonium, wenn man beispielsweise eine Mischung verschiedener Alkalimetallcyanide und/oder verschiedener Alkalimetall- bzw. Ammoniumsalze des β-Alanins einsetzt, oder Mono- oder Dialkalimetall- bzw. Ammoniumsalze der β-Alanindiessigsäure, wenn man entsprechend geringe Mengen an Mineralsäure einsetzt, erhalten werden.

Sollen β-Alanindiessigsäuresalze mit mehr als einem Ammoniumkation erhalten werden, geht man am besten so vor, daß man nach dem erfindungsgemäßen Verfahren hergestellte freie β-Alanindiessigsäure wieder mit der entsprechenden Menge an Ammoniak in wäßriger Lösung neutralisiert.

Das eingesetzte β-Alanin oder ein Alkalimetallsalz, insbesondere Natrium-oder Kaliumsalz, oder das Ammoniumsalz hiervon sollte eine Mindestreinheit von ca. 95 Gew.-%, vorzugsweise von 99 Gew.-%, aufweisen, da sonst die Reinheit des Endproduktes beeinträchtigt werden könnte. Derart reines β-Alanin bzw. Salze hiervon sind aber - auch im technischen Maßstab - gut zugänglich, ganz im Gegensatz zu vergleichbar reiner Iminodiessigsäure, welche das Ausgangsmaterial für die Synthese gemäß (1) darstellt.

Zweckmäßigerweise setzt man die Reaktionspartner β-Alanin bzw. sein Alkalimetallsalz, Alkalimetallcyanid, insbesondere Natrium- oder Kaliumcyanid, und Formaldehyd im notwendigen stöchiometrischen oder annähernd stöchiometrischen Verhältnis, d.h. 1 : 2 2, ein. Mann kann aber auch zum vollständigeren Umsatz des β-Alanins einen geringen Überschuß der weiteren Reaktionspartner, etwa bis zu 20 % in Kauf nehmen, ohne daß sich die Reinheit des Endproduktes wesentlich verschlechtert.

Formaldehyd wird vorteilhafterweise in Form seiner ca. 20 bis 35 gew.-%igen wäßrigen Lösung eingesetzt.

Die Umsetzung des β-Alanins bzw. seines Alkalimetall- oder Ammoniumsalzes mit Alkalimetallcyanid und Formaldehyd wird in der Regel bei erhöhten Temperaturen, zweckmäßigerweise bei 40 bis 100 °C, insbesondere 65 bis 90 °C, durchgeführt.

Man arbeitet hierbei vorteilhafterweise bei einem vermindertem Druck von 100 bis 800 mbar, insbesondere 400 bis 600 mbar.

Weiterhin ist es günstig, einen Luft- oder Inertgasstrom, z.B. Stickstoff, während der Umsetzung durch die Reaktionsmischung zu leiten und gebildetes Ammoniak in Form von Ammoniakwasser kontinuierlich abzudestillieren.

Es empfiehlt sich, die beiden Reaktionspartner Alkalimetallcyanid, welches in der Regel als wäßrige Lösung eingesetzt wird, und Formaldehyd über längere Zeit kontinuierlich und gleichzeitig dem vorgelegten β-Alanin zuzudosieren. Die Umsetzung ist so nach üblicherweise 4 bis 24 Stunden, insbesondere 6 bis 15 Stunden, abgeschlossen.

Der pH-Wert der wäßrigen Reaktionslösung liegt in der Regel bei 10 bis 14, insbesondere bei 12 bis 14. Zur Einstellung des pH-Wertes können die üblichen Basen wie Natriumhydroxid, Kaliumhydroxid, Natriumcarbonat oder Kaliumcarbonat verwendet werden. Setzt man ein Alkalimetallsalz des β-Alanins als Ausgangsmaterial ein, erübrigt sich meist eine zusätzliche Basenzugabe.

Gewünschtenfalls können die Alkalimetall- bzw. Ammoniumsalze I auf an sich bekannte Weise, z.B. durch Abdestillieren von Wasser und anschließende Kristallisation oder durch Sprühtrocknung, in fester Form erhalten werden.

Sollen ein, zwei oder vorzugsweise alle drei Carboxylgruppen von I als freie Säure vorliegen, werden diese durch Zugabe der entsprechenden Menge Mineralsäure aus ihren Salzen freigesetzt. Als Mineralsäuren können beispielsweise Schwefelsäure, Salzsäure, ortho-Phosphorsäure oder Salpetersäure eingesetzt werden. Als besonders günstig hat sich 20 bis 100 gew.-%ige, insbesondere 30 bis 70 gew.-%ige Schwefelsäure erwiesen.

Man arbeitet hierbei in der Regel bei Temperaturen von 10 bis 90 °C, vorzugsweise 20 bis 60 °C. Die freie Carboxylgruppen enthaltenden Verbindungen I werden zweckmäßigerweise durch Kristallisation oder Fällung isoliert. Soll β-Alanindiessigsäure mit drei freien Carboxylgruppen erhalten werden, fällt man das Produkt besonders vorteilhaft durch Zugabe von 30 bis 70 gew.-%iger Schwefelsäure bei einem pH-Wert von 2 bis 3,5, insbesondere 2,7 bis 3,1 aus. Es empfiehlt sich, die Fällung oder Kristallisation durch anschließendes Abkühlen auf Temperaturen von 0 bis 35 °C zu vervollständigen.

Das erfindungsgemäße Verfahren kann sowohl diskontinuierlich als auch kontinuierlich durchgeführt werden.

Gegenstand der vorliegenden Erfindung ist weiterhin die Verwendung der nach dem erfindungsgemäßen Verfahren hergestellten β-Alanindiessigsäure und ihrer Alkalimetallsalze I als Komplexbildner.

Das erfindungsgemäße Verfahren gestattet es, β-Alanindiessigsäure und ihre Alkalimetallsalze in hochreiner Form, d.h. mit höheren Gehalten als 99,5 Gew.-%, ohne zusätzliche Reinigungsschritte in einer einfachen und wirtschaftlichen Synthese auch im technischen Maßstab herzustellen.

Das erfindungsgemäße Verfahren liefert zudem keine störenden Nebenprodukte; anfallendes Ammoniakwasser kann leicht einer weiteren Verwertung zugeführt werden.

Beispiel

Herstellung von β-Alanindiessigsäure

Zu 1666 g einer 40 gew.-%igen wäßrigen Lösung des Natriumsalzes von β-Alanin (entsprechend 6,0 mol Natrium-β-alaninat) wurden bei 82 °C und 520 mbar innerhalb von 10 Stunden gleichzeitig 1200 g 30 gew.-%ige wäßrige Formaldehyd-Lösung (entsprechend 12,0 mol $CH_2O$) und 1980 g einer 30 gew.-%igen wäßrigen Natriumcyanid-Lösung (entsprechend 12,12 mol NaCN) getropft. Während der Reaktion wurde ein kräftiger Luftstrom durch das Reaktionsgemisch geleitet und es wurden kontinuierlich ca. 120 ml Ammoniakwasser pro Stunde abdestilliert. Der pH-Wert des Reaktionsgemisches lag während der Umsetzung bei 12,5 bis 13,5.

Nach vollständiger Zugabe von Formaldehyd und Natriumcyanid wurde die Temperatur auf 85 °C und der Druck auf 600 mbar erhöht und so lange weiter Ammoniakwasser abdestilliert, bis die $NH_3$-Konzentration im Reaktionsgemisch einen Wert von 0,01 Gew.-% erreichte.

Der Ansatz wurde mit ca. 750 g destilliertem Wasser verdünnt und bei 40 °C mit 1770 g 50 gew.-%iger wäßriger Schwefelsäure versetzt, wobei sich ein pH-Wert von 2,9 einstellte. Anschließend wurde auf 25 °C abgekühlt und 1 Stunde bei dieser Temperatur nachgerührt. Der ausgefallene Niederschlag wurde abfiltriert, mit Eiswasser gewaschen, bis im Waschwasser der Sulfatnachweis mit wäßriger Bariumchlorid-Lösung negativ ausfiel, und im Vakuum bei 60 °C getrocknet. Man erhielt 985 g der Titelverbindung, entsprechend einer Ausbeute von 80 %; das Produkt schmolz bei 194 - 196 °C (unter Zersetzung) und hatte eine Reinheit von 99,8 %.

**Patentansprüche**

1. Verfahren zur Herstellung von $\beta$-Alanindiessigsäure und ihren Alkalimetallsalzen der Formel I

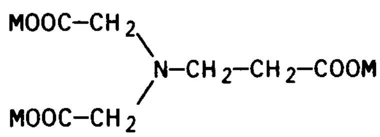

I

in der die Variablen M unabhängig voneinander für Wasserstoff oder Alkalimetall stehen, wobei auch eine Variable M für Ammonium stehen kann, dadurch gekennzeichnet, daß man $\beta$-Alanin oder ein Alkalimetallsalz oder das Ammoniumsalz hiervon mit Alkalimetallcyanid und Formaldehyd in wäßrig-alkalischem Medium umsetzt und für den Fall M = Wasserstoff die Carboxylgruppen durch Zugabe von Mineralsäure aus ihren Salzen freisetzt.

2. Verfahren nach Anspruch 1, dadurch gekennzeichnet, daß man die Umsetzung von $\beta$-Alanin oder einem Alkalimetallsalz oder dem Ammoniumsalz hiervon mit Alkalimetallcyanid und Formaldehyd bei Temperaturen von 40 bis 100 °C durchführt.

3. Verfahren nach Anspruch 1 oder 2, dadurch gekennzeichnet, daß man die Umsetzung von $\beta$-Alanin oder einem Alkalimetallsalz oder dem Ammoniumsalz hiervon mit Alkalimetallcyanid und Formaldehyd bei einem verminderten Druck von 100 bis 800 mbar durchführt.

4. Verfahren nach den Ansprüchen 1 bis 3, dadurch gekennzeichnet, daß man während der Umsetzung von $\beta$-Alanin oder einem Alkalimetallsalz oder dem Ammoniumsalz hiervon mit Alkalimetallcyanid und Formaldehyd einen Luft- oder Inertgasstrom durch die Reaktionsmischung leitet und gebildetes Ammoniak in Form von Ammoniakwasser kontinuierlich abdestilliert.

5. Verwendung von gemäß den Ansprüchen 1 bis 4 hergestellter $\beta$-Alanindiessigsäure und ihren Alkalimetallsalzen I als Komplexbildner.

**Patentansprüche für folgenden Vertragsstaat : ES**

1. Verfahren zur Herstellung von $\beta$-Alanindiessigsäure und ihren Alkalimetallsalzen der Formel I

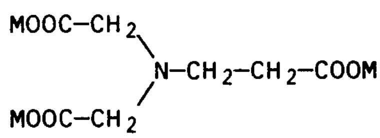

I

in der die Variablen M unabhängig voneinander für Wasserstoff oder Alkalimetall stehen, wobei auch eine Variable M für Ammonium stehen kann, dadurch gekennzeichnet, daß man $\beta$-Alanin oder ein Alkalimetallsalz oder das Ammoniumsalz hiervon mit Alkalimetallcyanid und Formaldehyd in wäßrig-alkalischem Medium umsetzt und für den Fall M = Wasserstoff die Carboxylgruppen durch Zugabe

von Mineralsäure aus ihren Salzen freisetzt.

2. Verfahren nach Anspruch 1, dadurch gekennzeichnet, daß man die Umsetzung von β-Alanin oder einem Alkalimetallsalz oder dem Ammoniumsalz hiervon mit Alkalimetallcyanid und Formaldehyd bei Temperaturen von 40 bis 100 °C durchführt.

3. Verfahren nach Anspruch 1 oder 2, dadurch gekennzeichnet, daß man die Umsetzung von β-Alanin oder einem Alkalimetallsalz oder dem Ammoniumsalz hiervon mit Alkalimetallcyanid und Formaldehyd bei einem verminderten Druck von 100 bis 800 mbar durchführt.

4. Verfahren nach den Ansprüchen 1 bis 3, dadurch gekennzeichnet, daß man während der Umsetzung von β-Alanin oder einem Alkalimetallsalz oder dem Ammoniumsalz hiervon mit Alkalimetallcyanid und Formaldehyd einen Luft- oder Inertgasstrom durch die Reaktionsmischung leitet und gebildetes Ammoniak in Form von Ammoniakwasser kontinuierlich abdestilliert.

5. Verfahren zum Komplexieren von Metallionen, dadurch gekennzeichnet, daß man hierbei gemäß den Ansprüchen 1 bis 4 hergestellte β-Alanindiessigsäure und ihre Alkalimetallsalze I als Komplexbildner verwendet.

Europäisches Patentamt

# EUROPÄISCHER RECHERCHENBERICHT

Nummer der Anmeldung

EP 91120734.8

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
| Y | DE - A - 2 427 898 (BASF) * Seite 4, Zeile 14 - Seite 5, Zeile 8 * | 1,2,4, 5 | C 07 C 229/16<br>C 07 C 227/26 |
| Y | CHEMICAL ABSTRACTS, Band 58, Nr. 5, 4. März 1963 Columbus, Ohio, USA N.F. KAZARINOVA et al. "Investigation of complexing agents derived from amino acids" Spalte 4644, Zusammenfassung-Nr. 4 644e & Tr. Soveshch. po Komplek-sonam, Akad. Nauk SSSR, Ural'sk. Filial, Sverdlovsk 1958, 39-52 | 1,2,4, 5 | |
| Y | DE - B - 2 625 974 (BASF) * Patentansprüche; Beispiel 1 * | 1-5 | RECHERCHIERTE SACHGEBIETE (Int. Cl.⁵) |
| D,Y | DE - A - 3 829 859 (BASF) * Seite 1, Zeilen 6-11; Ansprüche * | 1-5 | C 07 C 229/00<br>C 07 C 227/00 |
| D,A | CHEMICAL ABSTRACTS, BAnd 62, Nr. 13, 21. Juni 1965 Columbus, Ohio, USA V.G. YASHUNSKII et al. "Synthesis of some sydnonimine derivatives" Spalte 16232, Zusammen-fassung Nr. 16 232a & Zh. Vses. Khim. Obshchestva im.D.I. Mendeleeva 10(1), 150-6 | 1 | |

Der vorliegende Recherchenbericht wurde für alle Patentansprüche erstellt.

| Recherchenort | Abschlußdatum der Recherche | Prüfer |
|---|---|---|
| WIEN | 03-02-1992 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 62